# EUROPÄISCHE PATENTSCHRIFT

(11) **EP 0 971 886 B1**
(45) Veröffentlichungstag und Bekanntmachung des Hinweises auf die Patenterteilung: **15.01.2003**
(21) Anmeldenummer: 98913709.6
(22) Anmeldetag: 12.03.1998
(51) Int. Cl.: C07C 277/08, C07C 279/36

(54) **VERFAHREN ZUR HERSTELLUNG VON N-ALKYL-N'-NITROGUANIDINEN**
METHOD FOR PRODUCING N-ALKYL-N'-NITROGUANIDINES
PROCEDE DE PREPARATION DE N-ALKYLE-N'-NITROGUANIDINES

(30) Priorität: 27.03.1997 DE 19712885
(43) Veröffentlichungstag der Anmeldung: 19.01.2000
(73) Patentinhaber: Bayer CropScience AG, 40789 Monheim (DE)
(72) Erfinder: LANTZSCH, Reinhard, D-42115 Wuppertal (DE)
(86) Internationale Anmeldenummer: EP9801428
(87) Internationale Veröffentlichungsnummer: WO98043951

(56) Entgegenhaltungen:
- P.E. GAGNON ET AL.: "Alkylguanidine nitrates and alkylnitroguanidines" CAN. J. CHEM., Bd. 36, 1958, XP002069686 in der Anmeldung erwähnt

## Beschreibung

Die Erfindung betrifft ein neues Verfahren zur Herstellung von N-Alkyl-N'-nitroguanidinen.

Es ist bekannt, daß man N-Alkyl-N'-nitroguanidine erhält, wenn man zunächst S-Methylisothiuroniumsulfat der Formel (A) in üblicher Weise nitriert und anschließend in einer zweiten Reaktionsstufe die Methylmercaptogruppe gegen Alkylamin gemäß dem folgenden Reaktionsschema substituiert: 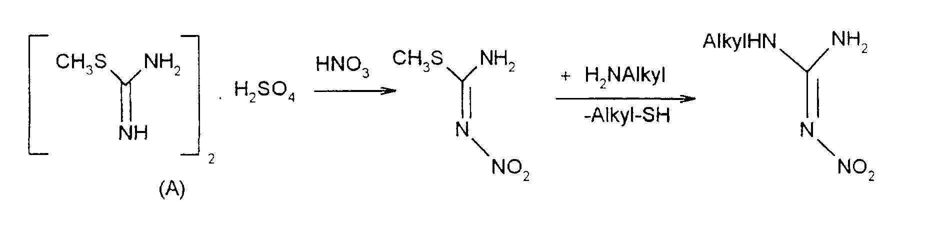 (vergl. hierzu JACS 76, 1877 (1954)).

Dieses Verfahren hat jedoch den Nachteil, daß es sich um eine zweistufige Umsetzung handelt. Wenngleich die Ausbeuten in beiden Stufen verhältnismäßig gut sind, wirft die Abspaltung von Alkylmercaptan, insbesondere von Methylmercaptan, vor allem bei einer Durchführung im großtechnischen Maßstab, verfahrenstechnische Probleme auf.

Ferner ist bekannt, daß N-Alkyl-, insbesondere N-Methyl-N'-nitroguanidine erhalten werden können, wenn man eine alkalische Lösung (Kaliumhydroxid) von Nitroguanidin mit Alkyl(insbesondere Methyl)amin-Hydrochlorid bei 60°C gemaß dem folgenden Reaktionsschema umsetzt:
z.B. 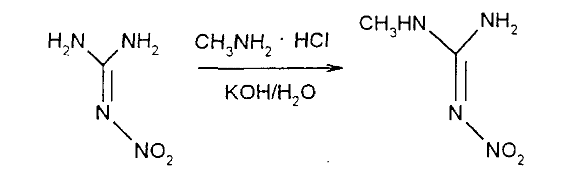 (vergl. hierzu JACS 69, 3028 (1947) bzw. 49, 2303 (1927)).

Bei diesem Prozeß sind die Ausbeuten und insbesondere die Reinheit der Endprodukte, die in aufwendigen Verfahren gereinigt werden müssen, sehr unbefriedigend. Als Nebenprodukte entstehen unter Entwicklung von gasförmigen N₂O größere Mengen Alkyl(Methyl)harnstoff. Hinzu kommen große Mengen an Abwasser und die Tatsache, daß Nitroguanidin ein relativ teurer Ausgangsstoff ist.

Außerdem ist bekannt, daß man N-Alkyl-N'-nitroguanidine durch Nitrierung von Alkylguanidinsulfaten erhalten kann (vergl. hierzu JACS, 55, 731 (1933)).

Auch diese Verfahrensvariante hat den Nachteil einer nicht zufriedenstellenden Ausbeute sowie einer relativ großen Abwassermenge.

Eine weitere bekannte Herstellungsmethode ist die Dehydratisierung von Alkylguanidinnitraten mittels Schwefelsäure (vergl. hierzu JACS, 55, 739 (1933)).

Dieses Verfahren verläuft zwar in verhältnismäßig guten Ausbeuten, doch ist die Zugänglichkeit der Ausgangsstoffe, wie beispielsweise des Methylguanidinnitrats sehr schwierig (vergl. hierzu Zeitschrift für angewandte Chemie 42, 380 (1929)).

Zur Herstellung des Nitrats muß Bariumnitrat verwendet werden, die Ausbeute beträgt nur 40 bis 50 %.

Auch die Umsetzung von Aminnitraten mit Calciumcyanamid oder Dicyandiamid verläuft mit unbefriedigenden Ausbeuten (Can. J. Chem. 36, 737-743 (1958)). Die beschriebenen Verfahren haben verschiedene Nachteile: Überschuß an Alkylamin, das nur schwierig wiedergewonnen werden kann, sowie (mit Dicyandiamid) hohe Verfahrenstemperaturen.

Es wurde nun gefunden, daß man N-Alkyl-N'-nitroguanidine der Formel (I) erhält, 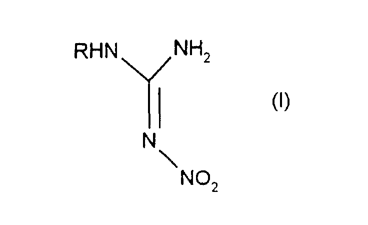 in welcher
- R: für C₁-C₄-Alkyl steht,
wenn man Alkylamin, gegebenenfalls in wäßriger Lösung und gegebenenfalls in Gegenwart eines organischen Lösungsmittel mit Salpetersäure neutralisiert, anschließend gegebenenfalls Wasser entfernt und mit Cyanamid in Gegenwart eines organischen Lösungsmittel und gegebenenfalls unter Druck bei Temperaturen zwischen 80°C und 180°C umsetzt und das gebildete Alkylguanidinnitrat dehydratisiert.

Überraschenderweise können nach dem erfindungsgemäßen Verfahren N-Alkyl-N'-nitroguanidine der Formel (I) auf einfache Art in sehr guten Ausbeuten und in hoher Reinheit erhalten werden, obwohl nach dem Stand der Technik zu erwarten war, daß bei Einsatz äquimolarer Mengen Amin (oder nur einem sehr geringen Überschuß) nur niedrige Ausbeuten erhalten werden sollten.

Schließlich war die Herstellung niedrig alkylsubstituierter Guanidinsalze aus Alkylaminsalzen und Cyanamid bei Temperaturen zwischen 170 bis 240°C bekannt (DOS 2 005 816). Es war somit vom Fachmann nicht vorhersehbar, daß diese Reaktion auch bei wesentlich tieferen Temperaturen durchführbar ist.

Die erfindungsgemäße Umsetzung hat die Vorteile, daß kein oder nur eine geringe Menge an überschüssigem Aminnitrat verwendet werden muß und das Verfahren bei geringerer Temperatur durchgeführt werden kann. Somit wird kostengünstiger, energiesparender und umweltschonender produziert.

Die nach dem erfindungsgemäßen Verfahren herstellbaren Verbindungen sind durch die Formel (I) allgemein definiert. In dieser Formel steht R vorzugsweise für Alkyl mit 1 bis 4 Kohlenstoffatomen, wie insbesondere für Methyl und Ethyl.

Verwendet man beispielsweise Methylamin als Ausgangsstoff, so kann der Reaktionsablauf des erfindungsgemäßen Verfahrens durch das folgende Formelschema skizziert werden:

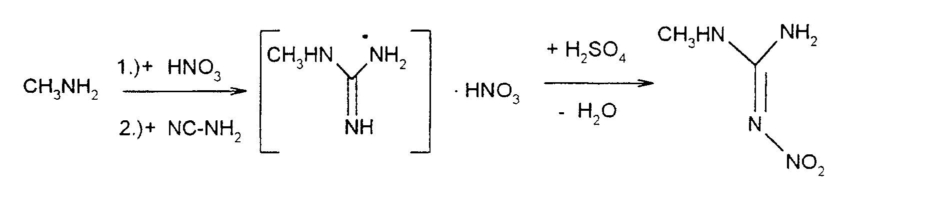

Die Ausgangsstoffe Alkylamin und Cyanamid sind allgemein bekannte Verbindungen der organischen Chemie.

Als organische Lösungsmittel kommen alle gegen die Reaktanden inerte organische Verdünnungsmittel infrage. Hierzu gehören vorzugsweise Alkohole, wie Methanol, Ethanol oder Butanol sowie Ether, wie Ethylenglykoldimethylether, Ethylenglykoldiethylether, Diethylenglykoldimethylether oder Methyl-tert-amylether.

Die Umsetzung mit Cyanamid wird vorzugsweise unter Sauerstoffausschluß in einem geschlossenen Gefäß durchgeführt, so daß sich ein Überdruck zwischen 1 und 20 bar, vorzugsweise 5 bis 15 bar ausbildet.

Die Reaktionstemperaturen können bei der Umsetzung mit Cyanamid in einem größeren Bereich variiert werden. Im allgemeinen arbeitet man bei Temperaturen zwischen 80°C und 180°C, vorzugsweise zwischen 100°C und 150°C.

Als Dehydratisierungsmittel kommen prinzipiell alle wasserabspaltenden Mittel infrage. Vorzugsweise genannt seien Sulfonsäuren, Schwefelsäure und Trifluoressigsäure.

Bei der Durchführung des erfindungsgemäßen Verfahrens arbeitet man vorzugsweise mit äquimolaren Mengen. Es ist aber auch möglich, mit einem Überschuß an Alkylamin ( 1 bis 1,5 Mol) zu arbeiten. Ein geringer Überschuß an Cyanamid (1 bis 1,2 Mol) ist ebenfalls möglich.

Die Aufarbeitung kann auf übliche Art und Weise durchgeführt werden.

Vor der Umsetzung des salpetersauren Salzes des Alkylamins mit Cyanamid wird bevorzugt Wasser z.B. destillativ azeotrop entfernt.

Die nach dem erfindungsgemäßen Verfahren herzustellenden N-Alkyl-N'-nitroguanidine der Formel (I), wie insbesondere das N-Methyl-N'-nitroguanidin, können als Zwischenprodukte zur Herstellung von biologisch aktiven Verbindungen, beispielsweise von Insektiziden verwendet werden (vergl. z.B. EP-A 0 376 279 und EP-A 0 428 941).

### Herstellungsbeispiele

### Beispiel 1

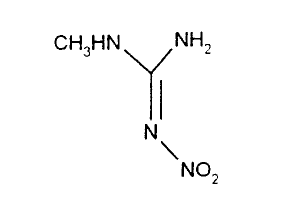

5,2 g (0,05 Mol) einer 30 %igen wässrigen Methylaminlösung wird mit ca. 4,2 g 75 %iger Salpetersäure neutralisiert und anschließend im Vakuum das Wasser abdestilliert.

Man fügt 15 ml n-Butanol und 2,2 g Cyanamid zu und befüllt damit einen 50 ml-Autoklaven. Anschließend erhitzt man 8 Stunden auf 130°C Innentemperatur. Nach dem Entspannen wird Butanol abdestilliert und der Rückstand unter Kühlung und Rühren vorsichtig mit 8,5 ml konzentrierter Schwefelsäure bei -5°C bis +5°C versetzt. Nach 30 Minuten werden 50 g Eis zugegeben, abfiltriert und mit wenig kaltem Wasser nachgewaschen.

Man erhält 5,5 g (93 % der Theorie) N-Methyl-N'-nitroguanidin vom Schmelzpunkt 160°C.

### Beispiel 2

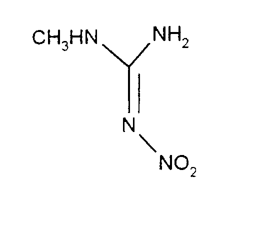

5,2 g (0,05 Mol) einer 30 %igen wäßrigen Methylaminlösung wird mit ca. 4,2 g 75 %iger Salpetersäure neutralisiert und anschließend im Vakuum das Wasser abdestilliert.

Man fügt 15 ml n-Butanol und 2,2 g Cyanamid zu und befüllt damit einen 50 ml-Autoklaven. Anschließend erhitzt man 8 Stunden auf 140°C Innentemperatur. Nach dem Entspannen kühlt man auf 0°C bis 5°C ab und filtriert. Der Filterkuchen wird getrocknet und unter Kühlung und Rühren vorsichtig mit 9,5 ml konzentrierter Schwefelsäure bei -5°C bis +5°C versetzt. Nach 20 Minuten werden 50 g Eis zugegeben, abfiltriert und mit wenig kaltem Wasser nachgewaschen.

Man erhält 5,3 g (89,6 % der Theorie) N-Methyl-N'-nitroguanidin vom Schmelzpunkt 160°C.

### Beispiel 3

Man tropft bei 0°C unter Kühlung zu 82,7 g (0,8 Mol) 30 %iger wässriger Methylaminlösung 100,8 g (0,8 Mol) 50 %ige Salptersäure, rührt 30 Minuten bei 0°C nach, läßt anschließend auf Raumtemperatur erwärmen und destilliert das Wasser ab. Danach gibt man 400 ml n-Butanol zu und destilliert solange, bis das restliche Wasser entfernt ist. Dann gibt man 30 g (0,72 Mol) Cyanamid zu und erhitzt in einem Autoklaven unter Rühren 8 h auf 130°C. Man kühlt ab, filtriert den ausgefallenen Feststoff ab, wäscht mit wenig n-Butanol nach und trocknet. Der Feststoff wird in 250 g, auf 0°C gekühlte, 98 %ige Schwefelsäure dosiert (innerhalb von 10 bis 15 Minuten). Anschließend wird auf 40°C erwärmt und untere Rühren 5 bis 10 Minuten auf dieser Temperatur gehalten. Dann kühlt man wieder auf 0°C ab und versetzt mit 750 g Eis. Der ausgefallene Feststoff wird abfiltriert und getrocknet. Man erhält 77,3 g (91 % der Theorie bezogen auf eingesetztes Cyanamid) Methylnitroguanidin (Gehalt lt. HPLC und Kapillarelektrophorese 100 %).

## Patentansprüche

1. Verfahren zur Herstellung von N-Alkyl-N'-nitroguanidinen der Formel (I) 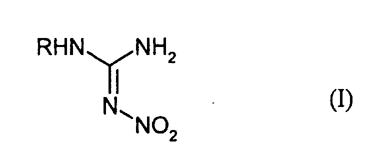 in welcher
R für C₁-C₄-Alkyl steht,
**dadurch gekennzeichnet, dass** man Alkylamin mit Salpetersäure neutralisiert, anschließend gegebenenfalls Wasser entfernt und mit Cyanamid in Gegenwart eines organischen Lösungsmittels bei Temperaturen zwischen 80°C und 180°C umsetzt und das gebildete Alkylguanidinnitrat dehydratisiert.

2. Verfahren gemäß Anspruch 1, **dadurch gekennzeichnet, dass** man die Umsetzung mit Cyanamid unter Druck, wasserfrei in einem inerten organischen Lösungsmittel durchführt.

3. Verfahren zur Herstellung von N-Alkyl-N'-nitroguanidinen der Formel (I), gemäß Anspruch 1, **dadurch gekennzeichnet, dass** man das gebildete Alkylguanidinnitrat mit einer Säure dehydratisiert.

## Claims

1. Process for the preparation of N-alkyl-N'-nitroguanidines of the formula (I) 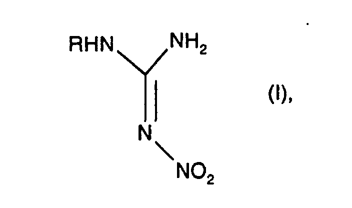 in which
R is C₁-C₄-alkyl,
**characterized in that** alkylamine is neutralized with nitric acid, then water is optionally removed and the mixture is reacted with cyanamide in the presence of an organic solvent at temperatures between 80°C and 180°C, and the alkylguanidine nitrate formed is dehydrated.

2. Process according to Claim 1, **characterized in that** the reaction with cyanamide is carried out under pressure without water in an inert organic solvent.

3. Process for the preparation of N-alkyl-N'-nitroguanidines of the formula (I) according to Claim 1, **characterized in that** the alkylguanidine nitrate formed is dehydrated using an acid.

## Revendications

1. Procédé de production de N-alkyl-N'-nitroguanidines de formule (I) 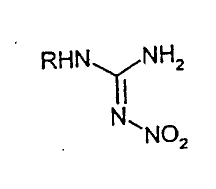 dans laquelle
R est un groupe alkyle en C₁ à C₄,
**caractérisé en ce qu'**on neutralise une alkylamine avec de l'acide nitrique, on élimine ensuite éventuellement l'eau et on fait réagir avec le cyanamide en présence d'un solvant organique à des températures comprises entre 80°C et 180°C et on déshydrate le nitrate d'alkylguanidine formé.

2. Procédé suivant la revendication 1, **caractérisé en ce qu'**on conduit la réaction avec le cyanamide sous pression, dans des conditions anhydres, dans un solvant organique inerte.

3. Procédé de production de N-alkyl-N'-nitroguanidines de formule (I) suivant la revendication 1, **caractérisé en ce qu'**on déshydrate avec un acide le nitrate d'alkyl-guanidine formé.
